# EUROPEAN PATENT SPECIFICATION

(11) **EP 1 299 130 B1**
(45) Date of publication and mention of the grant of the patent: **12.04.2006**
(21) Application number: 01940894.7
(22) Date of filing: 21.06.2001
(51) Int. Cl.: A61L 2/00, C12N 7/04

(54) **A METHOD OF TREATING AND PREVENTING INFECTIOUS DISEASES**
METHODE ZUR BEHANDLUNG UND PRÄVENTION VON ANSTECKENDEN KRANKHEITEN
METHODE DE TRAITEMENT ET DE PREVENTION DE MALADIES INFECTIEUSES

(30) Priority: 29.06.2000 AU PQ846900; 28.12.2000 WO PCT/AU00/01603
(43) Date of publication of application: 09.04.2003
(73) Proprietor: Lipid Sciences, Inc., Pleasanton, CA 94566 (US)
(72) Inventor: CHAM, Bill E., Sheldon, Queensland 4157 (AU)
(74) Representative: Dey, Michael
(86) International application number: PCT/IB2001/001099
(87) International publication number: WO 2002/000266

(56) References cited:
- EP-A- 0 222 974
- US-A- 4 522 809
- US-A- 4 895 558
- US-A- 5 698 432
- US-A- 5 744 038

## Description

### FIELD OF THE INVENTION

The present invention relates to a method for reducing the occurrence and severity of infectious diseases, especially infectious diseases in which infectious organisms are found in biological fluids, such as blood. The method of the present invention employs a system to treat infectious organisms which contain lipids. The present invention employs a solvent system useful for extracting lipids from the infectious organism, thereby reducing the infectivity of the infectious organism. The present invention also reduces the spread of infectious disease by providing a composition comprising a vaccine, comprising an infectious organism, treated with the method of the present invention to reduce the lipid content of the infectious organism, combined with a pharmaceutically acceptable carrier, and administered to an animal or a human.

### BACKGROUND OF THE INVENTION

Infectious disease is a major cause of suffering and death throughout the world. Infectious disease of varied etiology affects billions of animals and humans each year and inflicts an enormous economic burden on society. Many infectious organisms contain lipid as a major component of the membrane that surrounds them. Organisms which produce infectious disease and contain lipid in their cell wall or envelope include but are not limited to bacteria, viruses, protozoa, molds, and fungi. Numerous bacteria and viruses which affect animals and humans cause extreme suffering, morbidity and mortality. Many bacteria and viruses travel throughout the body in biological fluids, such as blood. These and other infectious organisms may be found in other biological fluids such as peritoneal fluid, lymphatic fluid, pleural fluid, pericardial fluid, cerebrospinal fluid, and in various fluids of the reproductive system. Disease may be caused at any site bathed by these fluids. Other bacteria and viruses reside primarily in different organ systems and in specific tissues, proliferate, and then enter the circulatory system to gain access to other tissues and organs at remote sites.

Infectious organisms, such as viruses, affect billions of people annually. Recent epidemics include the disease known as acquired immune deficiency syndrome (AIDS), believed to be caused by the human immunodeficiency virus (HIV). Related viruses affect other animals such as primates and cats. This virus is rapidly spreading throughout the world and is prevalent in various sub-populations of individuals, including individuals receiving blood transfusions, individuals using contaminated needles, and individuals having contact with infected biological fluids. This disease is also widespread in certain countries and affects more than one-third of the population. No known cure exists. What is needed is a simple, reliable and economic method for reducing the infectivity of the HIV virus so that transmission is decreased. What is also needed is a method to treat biological fluids of infected individuals in order to decrease transmission of the virus to others in contact with these biological fluids. What is also needed is a method to treat blood found in blood banks in order to decrease transmission of the virus through individuals receiving transfusions. Additionally what is needed for HIV as well as other viruses is a mechanism for decreasing the viral load of a human or an animal by treating the plasma of that individual and returning the treated plasma to the individual such that the viral load in the plasma is decreased.

Other major viral infections which affect animals and humans include, but are not limited to, meningitis, cytomegalovirus, and hepatitis in its various forms. While some forms of hepatitis may be treated with drugs, other forms are not successfully treated and are lethal. At the present time, most anti-viral therapies are directed to preventing or inhibiting viral replication and appear to focus on the initial attachment of the virus to the T4 lymphocyte or macrophage, the transcription of viral RNA to viral DNA and the assembly of new virus during reproduction. However, a major difficulty with existing treatments, especially with regard to HIV, is the high mutation rate of the virus. Many different strains of HIV are resistant or become resistant to anti-viral drug therapy. Furthermore, during anti-viral therapy treatment, resistant strains of the virus may evolve. Finally, many common therapies for HIV infection involve numerous undesirable side effects and require patient compliance with the ingestion of numerous pills every day or several times a day. Unfortunately, many individuals are afflicted with multiple infections caused by more than one infectious organism, such as HIV and hepatitis. Such individuals require even more aggressive and expensive drug regimens to counteract disease progression. Such regimens may cause numerous side effects as well as multi-drug resistance.

Prior art methods of inactivated viruses using chemical agents have relied on organic solvents such as chloroform. However, chloroform denatures many plasma proteins and is unsuitable for use with fluids which will subsequently be administered back to the animal or human. Many of the plasma proteins that are deleteriously affected by chloroform serve important biological functions including coagulation, hormonal responses, and immune responses. Many of these functions are essential to life, and so damage to proteins related to these functions may have an adverse effect on a patient's health, possibly leading to death. Other solvents such as B-propiolactone, detergents such as TWEEN-80, and di-or tri-alkyl phosphates have been used, alone or in combination. Many of these methods, especially those involving detergents, require tedious procedures to ensure removal of the detergent before reintroduction of the treated plasma sample into the animal or the human. Further, many of the methods described in the prior art involve extensive exposure to elevated temperature in order to kill free virus and infected cells. Numerous proteins contained in biological fluids such as plasma are deleteriously affected by elevated temperatures.

One such method is described in EP 0 222 974 A. EP 0 222 974 A concerns a process for the production of a rabies vaccine, wherein the inactivation of the virus is performed using beta-propiolactone (BLP) or tri-(n-butyl)phosphate. The overall method comprises obtaining rabies virus from suitable disintegrated tissue and extracting the crude virus suspension with a water-immiscible organic solvent for purification to remove foreign lipids, in particular, myelin. After purification and enrichment the intact viruses are inactivated by means of beta-propiolactone or tri-(n-butyl)phosphate. Accordingly, what is needed is a method which is simple, effective, does not require the use of elevated temperatures, and does not appreciably denature plasma proteins or extract them from the biological sample being treated.

Prevention of disease and amelioration of the severity of disease caused by infectious organisms is a major goal for modem medicine. Vaccination programs have reduced the occurrence and severity of many diseases although numerous diseases caused by infectious organisms remain without effective vaccines. Accordingly what is needed are new vaccine compositions for providing protection against infectious organisms.

### SUMMARY OF THE INVENTION

The present invention solves the problems described above by providing a simple, effective and efficient method for treating aqueous fluids containing lipid-containing infectious organisms. The method of the present invention is effective in reducing the concentration of an lipid-containing infectious organisms in a biological fluid. The present invention is also effective in producing a vaccine against the lipid-containing infectious organism by treating a biological fluid containing the infectious organism such that the organism is still present but no longer infectious. A lipid-containing infectious organism, treated in this manner in order to reduce its infectivity, is administered to a recipient, such as an animal or a human, together with a pharmaceutically acceptable carrier and optionally an immunostimulant, in order to provoke an immune response in the animal or human against antigens from the delipidated infectious organism.

The present invention contemplates treatment of infectious organisms containing a lipid component in their cell wall or envelope. Accordingly, numerous viruses and bacteria are included within the infectious organisms which may be treated with the method of the present invention. The method of the present invention for treating an aqueous fluid containing an infectious organism containing lipids comprises: obtaining a fluid containing the infectious organism; contacting the fluid with a first organic solvent capable of solubilizing the lipid in the infectious organism; and, separating a first phase containing the lipids from the infectious organism from a second phase wherein the second phase is substantially free of the lipids and contains reduced levels of the infectious organism. Fluids treated in this manner may optionally be reintroduced into the animal or human.

Fluids which may be treated with the method of the present invention include but are not limited to the following: plasma; serum; lymphatic fluid; cerebrospinal fluid; peritoneal fluid; pleural fluid; pericardial fluid; various fluids of the reproductive system including but not limited to semen, ejaculatory fluids, follicular fluid and amniotic fluid; cell culture reagents such as normal sera, such as fetal calf serum or serum derived from any other animal or human; and immunological reagents such as various preparations of antibodies and cytokines.

### Infectious Organisms Treated with the Present Invention

Infectious organisms which may be treated with the method of the present invention include infectious organisms containing lipid. Such infectious organisms include, but are not limited to, viruses and bacteria, provided the virus or bacteria contains lipid in the viral envelope or bacterial cell wall, respectively. The methods of the present invention reduce infectivity of infectious organisms and also provide vaccines against these organisms.

Viral infectious organisms which may be inactivated by the above system include, but are not limited to, the lipid-containing viruses of the following genuses: *Alphavirus* (alphaviruses), *Rubivurus* (rubella virus), *Flavivirus* (Flaviviruses), *Pestivirus* (mucosal disease viruses), (unnamed, hepatitis C virus), *Coronavirus,* (Coronaviruses), *Torovirus,* (toroviruses), *Arteivirus,* (arteriviruses), *Paramyxovirus,* (Paramyxoviruses), *Rubulavirus* (rubulavriuses), *Morbillivirus* (morbillivuruses), *Pneumovirinae* (the pneumoviruses), *Pneumovirus* (pneumoviruses), *Vesiculovirus* (vesiculoviruses), *Lyssavirus* (lyssaviruses), *Ephemerovirus* (ephemeroviruses), *Cytorhabdovirus* (plant rhabdovirus group A), *Nucleorhabdovirus* (plant rhabdovirus group B), *Filovirus* (filoviruses), *Influenzavirus A, B* (influenza A and B viruses), *Influenza virus C* (influenza C virus), (unnamed, Thogoto-like viruses), *Bunyavirus* (bunyaviruses), *Phlebovirus* (phleboviruses), *Nairovirus* (nairoviruses), *Hantavirus* (hantaviruses), *Tospovirus* (tospoviruses), *Arenavirus* (arenaviruses), unnamed mammalian type B retroviruses, unnamed, mammalian and reptilian type C retroviruses, unnamed, type D retroviruses, *Lentivirus* (lentiviruses), *Spumavirus* (spumaviruses), *Orthohepadnavirus* (hepadnaviruses of mammals), *Avihepadnavirus* (hepadnaviruses of birds), *Simplexvirus* (simplexviruses), *Varicellovirus* (varicelloviruses), *Betaherpesvirinae* (the cytomegaloviruses), *Cytomegalovirus* (cytomegaloviruses), *Muromegalovirus* (murine cytomegaloviruses), *Roseolovirus* (human herpes virus 6), *Gammaherpesvirinae* (the lymphocyte-associated herpes viruses), *Lymphocryptovirus* (Epstein-Bar-like viruses), *Rhadinovirus* (saimiri-ateles-like herpes viruses), *Orthopoxvirus* (orthopoxviruses), *Parapoxvirus* (parapoxviruses), *Avipoxvirus* (fowlpox viruses), *Capripoxvirus* (sheeppoxlike viruses), *Leporipoxvirus* (myxomaviruses), *Suipoxvirus* (swine-pox viruses), *Molluscipoxvirus* (molluscum contagiosum viruses), *Yatapoxvirus* (yabapox and tanapox viruses), Unnamed, African swine fever-like viruses, *Iridovirus* (small iridescent insect viruses), *Ranavirus* (front iridoviruses), *Lymphocystivirus* (lymphocystis viruses of fish) *Togaviridae, Flaviviridae, Coronaviridae, Enabdoviridae, Filoviridae, Paramyxoviridae, Orthomyxoviridae, Bunyaviridae, Arenaviridae, Retroviridae, Hepadnaviridae, Herpesviridae, Poxviridae,* and any other lipid-containing virus.

These viruses include the following human and animal pathogens: Ross River virus, fever virus, dengue viruses, Murray Valley encephalitis virus, tick-borne encephalitis viruses (including European and far eastern tick-borne encephalitis viruses, hepatitis A virus, hepatitis B virus, hepatitis C virus, human coronaviruses 229-E and OC43 and others (causing the common cold, upper respiratory tract infection, probably pneumonia and possibly gastroenteritis), human parainfluenza viruses 1 and 3, mumps virus, human parainfluenza viruses 2, 4a and 4b, measles virus, human respiratory syncytial virus, rabies virus, Marburg virus, Ebola virus, influenza A viruses and influenza B viruses, *Arenaviruss:* lymphocytic choriomeningitis (LCM) virus; Lassa virus, human immunodeficiency viruses 1 and 2, hepatitis B virus, Vaccinia, Subfamily: human herpes viruses 1 and 2, herpes virus B, Epstein-Barr virus), (smallpox) virus, Yellow fever virus, cowpox virus, poliovirus, Norwalk virus, molluscum contagiosum virus, and any other lipid-containing virus.

Preferred viruses to be treated with the method of the present invention include the various immunodeficiency viruses including but not limited to human (HIV), simian (SIV), feline (FIV), as well as any other form of immunodeficiency virus. Other preferred viruses to be treated with the method of the present invention include but are not limited to hepatitis in its various forms, especially hepatitis A, hepatitis B and hepatitis C. Another preferred virus treated with the method of the present invention involves the bovine pestivirus. It is to be understood that the present invention is not limited to the viruses provided in the list above. All viruses containing lipid, especially in their viral envelope, are included within the scope of the present invention.

Bacteria constitute another preferred class of infectious organisms which may be treated with the method of the present invention provided the bacteria contains lipid, preferably in its bacterial cell wall. Preferred bacteria to be treated with the method of the present invention include but are not limited to the following: *Staphylococcus; Streptococcus,* including *S. pyogenes; Enterococci; Bacillus, including Bacillus anthracis,* and *Lactobacillus; Listeria; Corynebacterium diphtheriae; Gardnerella* including *G. vaginalis; Nocardia; Streptomyces; Thermoactinomyces vulgaris; Treponema; Camplyobacter; Pseudomonas* including *P.aeruginosa; Legionella; Neisseria* including *N.gonorrhoeae* and *N.meningitides; Flavobacterium* including *F. meningosepticum* and *F. odoratum; Brucella; Bordetella* including *B. pertussis* and *B. bronchiseptica; Escherichia* including *E. coli; Klebsiella; Enterobacter; Serratia* including *S*. marcescens and *S. liquefaciens; Edwardsiella; Proteus* including *P. mirabilis* and *P. vulgaris; Streptobacillus; Rickettsiaceae* including *R. rickettsii; Chlamydia* including *C. psittaci* and *C. trachomatis; Mycobacterium* including *M. tuberculosis, M. intracellulare, M. fortuitum, M. laprae, M. avium, M. bovis, M. africanum, M. kansasii, M. intracellulare,* and *M. lepraemurium;* and *Nocardia,* and any other bacteria containing lipid in their membranes.

Other lipid-containing infectious organisms that may be treated with the method of the present invention include, but are not limited to, protozoa, molds, and fungi.

Accordingly, it is an object of the present invention to provide a method for treating a fluid in order to reduce or eliminate the infectivity of infectious organisms contained therein.

It is another object of the present invention to decrease the concentration of the infectious organism within the fluid.

Yet another object of the present invention is to decrease the infectivity of the infectious organism contained within the fluid.

Still another of the present invention is to use the present method to decrease the concentration and infectivity of infectious organisms contained within a fluid.

It is a specific object of the present invention to decrease the infectivity of infectious organisms contained within a fluid wherein the fluid is plasma.

It is another specific object of the present invention to provide a method to reduce the infectivity and viral load of viruses found within a fluid such as plasma.

Yet another object of the present invention is to completely or partially inactivate and reduce the viral load of viruses contained within a sample such as plasma, wherein the viruses are human immunodeficiency virus, hepatitis in its various forms, or another virus.

Yet another object of the present invention is to reduce the infectivity and concentration of bacteria contained within a fluid, such as plasma.

It is further an object of the present invention to treat infectious organisms with the method of the present invention in order to reduce their infectivity and provide a vaccine comprising a delipidated infectious organism which may be administered to an animal or a human together with a pharmaceutically acceptable carrier and optionally an immunostimulant compound, to prevent or minimize clinical manifestation of disease following exposure to the infectious organism.

It is another specific object of the present invention to provide an anti-viral vaccine.

Yet another object of the present invention is to provide an antibacterial vaccine.

It is a further specific object of the present invention to confer immunity to a lipid-containing infectious organism in an animal or human receiving a vaccine comprising a composition comprising an infectious organism treated with the method of the present invention in a pharmaceutically acceptable carrier.

It is another object of the present invention to provide a method useful for development of vaccines against infectious organisms, including but not limited to, viruses and bacteria.

Yet another object of the present invention is to provide a solution containing inactivated viral particles from a treated lipid-containing virus that may be lyophilized and reconstituted when desired for administration to an animal or human.

It is further an object of the present invention to provide a method for treatment of lipid-containing infectious organisms within a fluid which minimizes deleterious effects on proteins contained within the fluid.

It is another object of the present invention to provide a method for reducing the infectivity of lipid-containing infectious organisms, wherein the method does not employ elevated temperatures, chloroform, detergents, or trialkyl phosphates.

These and other objects, advantages, and uses of the present invention will reveal themselves to one of ordinary skill in the art after reading the detailed description of the preferred embodiments and the attached claims.

### DETAILED DESCRIPTION OF THE INVENTION

### Definitions

By the term "fluid" is meant any fluid containing an infectious organism, including but not limited to, a biological fluid obtained from an organism such as an animal or human. Such biological fluids obtained from an organism include but are not limited to plasma, serum, cerebrospinal fluid, lymphatic fluid, peritoneal fluid, follicular fluid, amniotic fluid, pleural fluid, pericardial fluid, reproductive fluids and any other fluid contained within the organism. Other fluids may include laboratory samples containing infectious organisms suspended in any chosen fluid. Other fluids include cell culture reagents, many of which include biological compounds such as fluids obtained from living organisms, including but not limited to "normal serum" obtained from various animals and used as growth medium in cell and tissue culture applications.

By the term "first solvent" or "first organic solvent" is meant a solvent, comprising one or more solvents, that facilitates extraction of lipid.

By the term "demulsifying agent" is meant an agent that assists in the removal of the first solvent which may be present in an emulsion in an aqueous layer.

The terms "pharmaceutically acceptable carrier or pharmaceutically acceptable vehicle" are used herein to mean any liquid including but not limited to water or saline, a gel, salve, solvent, diluent, fluid ointment base, liposome, micelle, giant micelle, and the like, which is suitable for use in contact with living animal or human tissue without causing adverse physiological responses, and which does not interact with the other components of the composition in a deleterious manner.

By the term "infectious organism" is meant any lipid-containing infectious organism capable of causing infection. Some infectious organisms include bacteria, viruses, protozoa, parasites, fungi and mold. Some bacteria which may be treated with the method of the present invention include, but are not limited to the following: *Staphylococcus; Streptococcus,* including *S. pyogenes; Enterococci; Bacillus, including Bacillus anthracis,* and *Lactobacillus; Listeria; Corynebacterium diphtheriae; Gardnerella* including *G. vaginalis; Nocardia; Streptomyces; Thermoactinomyces vulgaris; Treponema; Camplyobacter; Pseudomonas* including *P.aeruginosa; Legionella; Neisseria* including *N.gonorrhoeae* and *N.meningitides; Flavobacterium* including *F. meningosepticum* and *F. odoratum; Brucella; Bordetella* including *B. pertussis,* and *B. bronchiseptica; Escherichia* including *E. coli; Klebsiella; Enterobacter; Serratia* including *S. marcescens* and *S. liquefaciens; Edwardsiella; Proteus* including *P. mirabilis* and *P. vulgaris; Streptobacillus; Rickettsiaceae* including *R. rickettsii; Chlamydia* including *C. psittaci* and *C. trachomatis; Mycobacterium* including *M. tuberculosis, M. intracellulare, M. fortuitum, M. laprae, M. avium, M. bovis, M. africanum, M. kansasii, M. intracellulare,* and *M. lepraemurium;* and *Nocardia,* and any other bacteria containing lipid in their membranes.

Viral infectious organisms which may be inactivated by the above system include, but are not limited to the lipid-containing viruses of the following genuses: *Alphavirus* (alphaviruses), *Rubivurus* (rubella virus), *Flavivirus* (Flaviviruses), *Pestivirus* (mucosal disease viruses), (unnamed, hepatitis C virus), *Coronavirus,* (Coronaviruses), *Torovirus,* (toroviruses), *Arteivirus,* (arteriviruses), *Paramyxovirus,* (Paramyxoviruses), *Rubulavirus* (rubulavriuses), *Morbillivirus* (morbillivuruses), *Pneumovirinae* (the pneumoviruses), *Pneumovirus* (pneumoviruses), *Vesiculovirus* (vesiculoviruses), *Lyssavirus* (lyssaviruses), *Ephemerovirus* (ephemeroviruses), *Cytorhabdovirus* (plant rhabdovirus group A), *Nucleorhabdovirus* (plant rhabdovirus group B), *Filovirus* (filoviruses), *Influenzavirus A, B* (influenza A and B viruses), *Influenza virus C* (influenza C virus), (unnamed, Thogoto-like viruses), *Bunyavirus* (bunyaviruses), Phlebovirus (phleboviruses), *Nairovirus* (nairoviruses), *Hantavirus* (hantaviruses), *Tospovirus* (tospoviruses), *Arenavirus* (arenaviruses), unnamed mammalian type B retroviruses, unnamed, mammalian and reptilian type C retroviruses, unnamed, type D retroviruses, *Lentivirus* (lentiviruses), *Spumavirus* (spumaviruses), *Orthohepadnavirus* (hepadnaviruses of mammals), *Avihepadnavirus* (hepadnaviruses of birds), *Simplexvirus* (simplexviruses), *Varicellovirus* (varicelloviruses), *Betaherpesvirinae* (the cytomegaloviruses), *Cytomegalovirus* (cytomegaloviruses), *Muromegalovirus* (murine cytomegaloviruses), *Roseolovirus* (human herpes virus 6), *Gammaherpesvirinae* (the lymphocyte-associated herpes viruses), *Lymphocryptovirus* (Epstein-Bar-like viruses), *Rhadinovirus* (saimiri-ateles-like herpes viruses), *Orthopoxvirus* (orthopoxviruses), *Parapoxvirus* (parapoxviruses), *Avipoxvirus* (fowlpox viruses), *Capripoxvirus* (sheeppoxlike viruses), *Leporipoxvirus* (myxomaviruses), *Suipoxvirus* (swine-pox viruses), *Molluscipoxvirus* (molluscum contagiosum viruses), *Yatapoxvirus* (yabapox and tanapox viruses), Unnamed, African swine fever-like viruses, I*ridovirus* (small iridescent insect viruses), *Ranavirus* (front iridoviruses), *Lymphocystivirus* (lymphocystis viruses of fish), *Togaviridae, Flaviviridae, Coronaviridae, Enabdoviridae, Filoviridae, Paramyxoviridae, Orthomyxoviridae, Bunyaviridae, Arenaviridae, Retroviridae, Hepadnaviridae, Herpesviridae, Poxviridae,* and any other lipid-containing virus.

These viruses include the following human and animal pathogens: Ross River virus, fever virus, dengue viruses, Murray Valley encephalitis virus, tick-borne encephalitis viruses (including European and far eastern tick-borne encephalitis viruses, hepatitis C virus, human coronaviruses 229-E and OC43 and others (causing the common cold, upper respiratory tract infection, probably pneumonia and possibly gastroenteritis), human parainfluenza viruses 1 and 3, mumps virus, human parainfluenza viruses 2, 4a and 4b, measles virus, human respiratory syncytial virus, rabies virus, Marburg virus, Ebola virus, influenza A viruses and influenza B viruses, *Arenaviruss:* lumphocytic choriomeningitis (LCM) virus; Lassa virus, human immunodeficiency viruses 1 and 2, or any other immunodeficiency virus, hepatitis A virus, hepatitis B virus, hepatitis C virus, Subfamily: human herpes viruses 1 and 2, herpes virus B, Epstein-Barr virus), (smallpox) virus, cowpox virus, molluscum contagiosum virus.

### Solvents for Use in Removal of Lipid from Lipid-Containing Organisms, Especially Infectious Organisms

Numerous organic solvents may be used in the method of the present invention for removal of lipid from lipid-containing organisms, especially infectious organisms, provided that the solvents or combinations thereof are effective in solubilizing lipids. Suitable solvents comprise mixtures of hydrocarbons, ethers, alcohols and amines. Other solvents which may be used with the present invention include amines and mixtures of amines. Preferred solvents are combinations of alcohols and ethers. Another preferred solvent comprises an ether or combinations of ethers. It is preferred that the solvent or combination of solvents has a relatively low boiling point to facilitate removal through a combination of vacuum and possibly heat.

Examples of suitable amines for use in removal of lipid from lipid-containing organisms in the present invention are those which are substantially water immiscible. Typical amines are aliphatic amines having a carbon chain of at least 6 carbon atoms. A non-limiting example of such an amine is C₆H₁₃NH₂.

The alcohols which are preferred for use in the present invention, when used alone, include those alcohols which are not appreciably miscible with plasma or other biological fluids. Such alcohols include, but are not limited to, straight chain and branched chain alcohols, including pentanols, hexanols, heptanols, octanols and alcohols containing higher numbers of carbons.

When alcohols are used in combination with another solvent, for example, an ether, a hydrocarbon, an amine, or a combination thereof, C₁₋C₈ containing alcohols may be used. Preferred alcohols for use in combination with another solvent include C₄-C₈ containing alcohols. Accordingly, preferred alcohols that fall within the scope of the present invention are preferably butanols, pentanols, hexanols, heptanols and octanols, and iso forms thereof. Particularly preferred are the butanols (1-butanol and 2-butanol). As stated above, the most preferred alcohol is the C₄ alcohol, butanol. The specific choice of alcohol will depend on the second solvent employed. In a preferred embodiment, lower alcohols are combined with lower ethers.

Ethers, used alone, or in combination with other solvents, preferably alcohols, are another preferred solvent for use in the method of the present invention. Particularly preferred are the C₄-C₈ containing-ethers, including but not limited to, ethyl ether, diethyl ether, and propyl ethers, including but not limited to di-isopropyl ether. Also useful in the present invention are combinations of ethers, such as di-isopropyl ether and diethyl ether. When ethers and alcohols are used in combination as a first solvent for contacting the fluid containing the lipid-containing organism infectious organism, any combination of alcohol and ether may be used provided the combination is effective to partially or completely remove lipid from the infectious organism. In one embodiment lipid is removed from the viral envelope or bacterial cell wall of the infectious organism. When alcohols and ether are combined as a first solvent for treating the infectious organism contained in a fluid, preferred ratios of alcohol to ether in this solvent are about 0.01%-60% alcohol to about 40%-99.99% of ether, with a preferred ratio of about 10%-50% of alcohol with about 50%-90% of ether, with a most preferred ratio of about 20%-45% alcohol and about 55%-80% ether. An especially preferred combination of alcohol and ether is the combination of butanol and di-isopropyl ether. Another especially preferred combination of alcohol and ether is the combination of butanol with diethyl ether. When butanol and di-isopropyl ether are combined as a first solvent for treating the infectious organism contained in a fluid, preferred ratios of butanol to di-isopropyl ether in this solvent are about 0.01 %-60% butanol to about 40%-99.99% of di-isopropyl ether, with a preferred ratio of about 10%-50% of butanol with about 50%-90% of di-isopropyl ether, with a most preferred ratio of about 20%-45% butanol and about 55%-80% di-isopropyl ether. The most preferred ratio of butanol and di-isopropyl ether is about 40% butanol and about 60% di-isopropyl ether.

When butanol is used in combination with diethyl ether in a first solvent, preferred ratios of butanol to diethyl ether in this combination are about 0.01%-60% butanol to about 40%-99.99% of diethyl ether, with a preferred ratio of about 10%-50% of butanol with about 50%-90% of diethyl ether, with a most preferred ratio of about 20%-45% butanol and about 55%-80% diethyl ether. The most preferred ratio of butanol and diethyl ether in a first solvent is about 40% butanol and about 60% diethyl ether. This combination of about 40% butanol and about 60% diethyl ether (vol:vol) has been shown to have no significant effect on a variety of biochemical and hematological blood parameters, as shown for example in U.S. Patent 4,895,558. Further comparisons were made on the serum pH, protein and enzyme activities in human serum when treated with butanol-DIPE (40%-60% V/V). The results are illustrated in the following table.

**TABLE 1**

| | | **Control** | **Delipidated** |
|---|---|---|---|
| IgA | mg/100ml | 168 | 167 |
| Igm | mg/100ml | 144 | 144 |
| Ceruloplasmin | mg/100ml | 1402 | 1395 |
| Transferrin | mg/100ml | 30 | 31 |
| Albumin | g/100ml | 5.12 | 5.12 |
| Total protein | g/100ml | 7.35 | 7.42 |
| pH | | 7.37 | 7.37 |
| GOT | IU | 25 | 23 |
| Alkaline phosphatase | IU | 81 | 80 |
| a-amylase | IU | 293 | 293 |

This solvent system of butanol-DIPE (40%-60% VN) does not adversely affect the blood constituents shown in the table above. Also, there appears to be little or no denaturation of plasma proteins or changes in enzyme activity, including the activity of lipid associated enzymes such as lecithin cholesterol acetyltransferase and cholesterol ester transfer protein.

### Solvents for Use in Vaccine Production

Different solvents and combinations of solvents may be used for treating an lipid-containing organism, such as an infectious organism, for producing a vaccine using the treated organism. This section describes these solvents and combinations thereof. Suitable solvents comprise hydrocarbons, ethers, alcohols, amines, surfactants, esters and combinations thereof.

Hydrocarbons in their liquid form dissolve compounds of low polarity such as the lipids found in membranes of infectious organisms. Hydrocarbons which are liquid at about 37°C are effective in disrupting a lipid membrane of an infectious organism. Accordingly, hydrocarbons comprise any substantially water immiscible hydrocarbon which is liquid at about 37°C. Suitable hydrocarbons include, but are not limited to the following: C₅ to C₂₀ aliphatic hydrocarbons such as petroleum ether, hexane, heptane, octane; haloaliphatic hydrocarbons such as chloroform, 1,1,2-trichloro-1,2,2-trifluoroethane, 1,1,1-trichloroethane, trichloroethylene, tetrachloroethylene dichloromethane and carbon tetrachloride, and thioaliphatic hydrocarbons each of which may be linear, branched or cyclic, saturated or unsaturated; aromatic hydrocarbons such as benzene; alkylarenes such as toluene, haloarenes, haloalkylarenes and thioarenes. Other suitable solvents may also include saturated or unsaturated heterocyclic compounds such as pyridine and aliphatic, thio or halo derivatives thereof.

Suitable esters which may be used include, but are not limited to, ethyl acetate, propylacetate, butylacetate and ethylpropionate.

Suitable surfactants which may be used, include but are not limited to the following: sulfates, sulfonates, phosphates (including phospholipids), carboxylates, and sulfosuccinates. Some anionic amphiphilic materials useful with the present invention include but are not limited to the following: sodium dodecyl sulfate (SDS), sodium decyl sulfate, bis-(2-ethylhexyl) sodium sulfosuccinate (AOT), cholesterol sulfate and sodium laurate.

The alcohols which are preferred for use in the present invention, when used alone, include those alcohols which are not appreciably miscible with plasma or other biological fluids. When alcohols are used in combination with another solvent, for example, ether, a hydrocarbon, an amine or a combination thereof, C₁-C₈ containing alcohols may be used. Preferred alcohols for use in combination with another solvent include lower alcohols such as C₄-C₈ containing alcohols. Accordingly, preferred alcohols that fall within the scope of the present invention are preferably butanols, pentanols, hexanols, heptanols and octanols, and iso forms thereof. Particularly preferred are the butanols (1-butanol and 2-butanol). As stated above, the most preferred alcohol is the C₄ alcohol, butanol. The specific choice of alcohol will depend on the second solvent employed. In a preferred embodiment, lower alcohols are combined with lower ethers.

Ethers, used alone, or in combination with other solvents, preferably alcohols, are another preferred solvent for use in the method of the present invention. Particularly preferred are the C₄-C₈ ethers, including but not limited to, ethyl ether, diethyl ether, and propyl ethers, including but not limited to di-isopropyl ether. Also useful in the present invention are combinations of ethers, such as di-isopropyl ether and diethyl ether.

When ethers and alcohols are used in combination as a first solvent for removing lipid from the infectious organism in order to make a vaccine, any combination of alcohol and ether may be used provided the combination is effective to partially or completely remove lipid from the infectious organism. In one embodiment lipid is removed from the viral envelope or bacterial cell wall of the infectious organism. When alcohols and ether are combined as a first solvent for treating the infectious organism contained in a fluid, preferred ratios of alcohol to ether in this solvent are about 0.01%-60% alcohol to about 40%-99.99% ether, with a preferred ratio of about 10%-50% alcohol with about 50%-90% ether, with a most preferred ratio of about 20%-45% alcohol and about 55%-80% ether. An especially preferred combination of alcohol and ether is the combination of butanol and di-isopropyl ether. Another especially preferred combination of alcohol and ether is the combination of butanol with diethyl ether. When butanol and di-isopropyl ether are combined as a first solvent for treating the infectious organism contained in a fluid, preferred ratios of butanol to di-isopropyl ether in this solvent are about 0.01%-60% butanol to about 40%-99.99% di-isopropyl ether, with a preferred ratio of about 10%-50% butanol with about 50%-90% di-isopropyl ether, with a most preferred ratio of about 20%-45% butanol and about 55%-80% di-isopropyl ether. The most preferred ratio of butanol and di-isopropyl ether is about 40% butanol and about 60% di-isopropyl ether.

When butanol is used in combination with diethyl ether in a first solvent, preferred ratios of butanol to diethyl ether in this combination are about 0.01%-60% butanol to about 40%-99.99% diethyl ether, with a preferred ratio of about 10%-50% butanol with about 50%-90% diethyl ether, with a most preferred ratio of about 20%-45% butanol and about 55%-80% diethyl ether. The most preferred ratio of butanol and diethyl ether in a first solvent is about 40% butanol and about 60% diethyl ether.

### Biological Fluids and Treatment Thereof for Reducing Infectivity of Infectious, Lipid-Containing Organisms

As stated above, various biological fluids may be employed with the method of the present invention in order to reduce the levels or infectivity of the lipid-containing organism in the fluid. In a preferred embodiment of the present invention, plasma obtained from an animal or human is treated with the method of the present invention in order to reduce the concentration and/or infectivity of lipid-containing infectious organisms within the plasma. In this embodiment, plasma may be obtained from an animal or human by withdrawing blood from the animal or human using known methods and treating the blood with conventional methods in order to separate the cellular components of the blood (red and white cells) from the plasma. Such methods are known to one of ordinary skill in the art and include centrifugation and filtration.

Viruses are typically retained in the plasma and are affected by the treatment of the plasma with the method of the present invention. When the lipid-containing organism to be treated is substantially larger than a virus, and may pellet with red and white blood cells under typical centrifugation conditions for separating cells from plasma, the lipid-containing organism may be separated from the red and white cells using techniques known to one of ordinary skill in the art. Such methods include but are not limited to centrifugation and filtration. One of ordinary skill in the art understands the proper centrifugation conditions for separating such lipid-containing organisms from the red and white cells. Filtration may include diafiltration or filtration through membranes with pore sizes that separate the lipid-containing organism, such as a bacteria from the red cells and white cells. Use of the present invention permits treatment of lipid-containing organisms, such as a bacteria, found within plasma, without deleterious effects on other plasma proteins.

Treatment of lipid-containing organisms in biological fluids other than blood and plasma does not generally involve separation of the cells from the fluid before the delipidation procedure is initiated. For example, follicular fluid and peritoneal fluid may be treated with the present invention to affect the levels and infectivity of lipid-containing organisms without deleterious effects on protein components. The treated fluid may then be returned to an animal or human. Treatment of these non-blood types of fluids affects the lipid-containing organisms in the fluid, including the bacteria and viruses.

Once a biological fluid, such as plasma, is obtained either in this manner, or for example, from a storage facility housing bags of plasma, the plasma is contacted with a first organic solvent as described above which is capable of solubilizing lipid in the lipid-containing infectious organism. The first organic solvent is combined with the plasma in a ratio wherein the first solvent is present in an amount effective to substantially solubilize the lipid in the infectious organism. Preferred ratios of first solvent to plasma (expressed as first organic solvent:plasma) are described in the following ranges: 0.5 - 4.0:0.5 - 4.0; 0.8 - 3.0:0.8 - 3.0; and 1-2:0.8-1.5.

It is to be understood that various other ratios may be employed for different fluids such as those fluids described above. For example, in the case of cell culture fluid, the following ranges may be employed of first organic solvent to cell culture fluid: 0.5 - 4.0:0.5 - 4.0; 0.8 - 3.0:0.8 - 3.0; and 1-2:0.8-1.5.

After contacting the fluid containing the infectious organism with the first solvent as described above, the first solvent and fluid are mixed. Suitable mixing methods include but are not limited to the following: gentle stirring; vigorous stirring; vortexing; swirling; homogenization; and end-over-end rotation.

The amount of time required for adequate mixing of the first solvent with the fluid is related to the mixing method employed. Fluids are mixed for a period of time sufficient to permit intimate contact between the organic and aqueous phases, and for the first solvent to solubilize some or all of the lipid contained in the infectious organism. Typically, mixing will occur for a period of about 10 seconds to about 24 hours, preferably about 10 seconds to about 2 hours, more preferably approximately 10 seconds to approximately 10 minutes, or about 30 seconds to about 1 hour, depending ion the mixing method employed. Non-limiting examples of mixing durations associated with different methods are presented in the next sentences. Gentle stirring and end-over-end rotation may occur for a period of about 10 seconds to about 24 hours. Vigorous stirring and vortexing may occur for a period of about 10 seconds to about 30 minutes. Swirling may occur for a period of about 10 seconds to about 2. hours. Homogenization may occur for a period of about 10 seconds to about 10 minutes.

Following mixing of the first solvent with the fluid, the solvent is separated from the fluid being treated. The separation may occur by any suitable manner known to one of ordinary skill in the art of separating organic and aqueous phases. Since the first solvent is typically immiscible in the aqueous fluid, separation is usually achieved by permitting the two layers to separate and removing the undesired layer. The undesired layer is the solvent layer containing dissolved lipids and depends on whether the solvent is more or less dense than the aqueous phase. An advantage of separation in this manner is that dissolved lipids in the solvent layer may be removed. Separation may be achieved through means, including but not limited to the following: removing the layer by pipetting; centrifugation followed by removal of the layer to be separated; creating a path or hole in the bottom of the tube containing the layers and permitting the lower layer to pass through; utilization of a container with valves or ports located at specific lengths along the long axis of the container to facilitate access to and removal of specific layers; and any other means known to one of ordinary skill in the art. Another method of separating the layers, especially when the solvent layer is volatile, is through distillation under reduced pressure or evaporation at room temperature, optionally combined with mild heating. In one embodiment employing centrifugation, relatively low g forces are employed, such as 900 x g for about 5 to 15 minutes to separate the phases.

Following separation of the first solvent from the treated fluid, some of the first solvent may remain entrapped in the aqueous layer. This may be in the form of an emulsion. Optionally, a de-emulsifying agent is employed to facilitate removal of the trapped first solvent. The de-emulsifying agent may be any agent effective to facilitate removal of the first solvent. A preferred de-emulsifying agent is ether and a more preferred de-emulsifying agent is diethyl ether. The de-emulsifying agent may be added to the fluid or alternatively, the fluid may be dispersed in the de-emulsifying agent. When a vaccine is prepared, alkanes in a ratio of about 0.5 to 4.0 to about 1 part of emulsion (vol:vol) may be employed as a demulsifying agent, followed by washing to remove residual alkane from the delipidated organism used for preparing the vaccine. Preferred alkanes include, but are not limited to, pentane, hexane and higher order straight and branched chain alkanes.

The de-emulsifying agent, such as ether, may be removed through means known to one of skill in the art, including such means as described in the previous paragraph. One convenient method to remove the de-emulsifying agent, such as ether, from the system, is to permit the ether to evaporate from the system in a fume hood or other suitable device for collecting and removing the de-emulsifying agent from the environment. De-emulsifying agents may be removed through application of higher temperatures, for example from about 24 to 37°C with or without pressures of about 10 to 20 mbar. Another method to remove the de-emulsifying agent involves separation by centrifugation, removal of organic solvent through aspiration followed by evaporation under reduced pressure (for example 50 mbar) or further supply of an inert gas, such as nitrogen, over the meniscus.

It is to be understood that the method of the present invention may be employed in a continuous or discontinuous manner. That is, in a continuous manner, a fluid may be fed in a continuous manner to a system employing a first solvent which is then mixed with the fluid, separated, and optionally further removed through application of a de-emulsifying agent. The continuous method also facilitates subsequent return of the fluid containing delipidated infectious organism to a desired location. Such locations may be containers for receipt and/or storage of such treated fluid, and may also include the vascular system of a human or animal or some other body compartment of a human or animal, such as the pleural, pericardial, peritoneal, and abdominopelvic spaces. For example, in one embodiment of the present invention, the method may be used continuously in the following scenario. A biological fluid, for example blood, is removed from an animal or a human through means known to one of ordinary skill in the art, such as a catheter. Appropriate anti-clotting factors as known to one of skill in the are employed, such as heparin, ethylenediaminetetraacetic acid (EDTA) or citrate. This blood is then separated into its cellular and plasma components through the use a centrifuge. The plasma is then contacted with the first solvent and mixed with the first solvent to effect lipid removal from the infectious organism contained within the plasma. Following separation of the first solvent from the treated plasma, a de-emulsifying agent is optionally employed to remove entrapped first solvent. After ensuring that acceptable levels of first solvent or de-emulsifying agent, if employed, are found within the plasma containing the delipidated infectious organism, the plasma is then optionally combined with the cells previously separated from the blood to form a new blood sample containing partially or completely delipidated infectious organisms. In any event, the infectivity of the infectious organism is greatly reduced or eliminated through the method of the present invention. Following re-combination with the cells originally separated from the blood, this sample may be reintroduced into the vascular system or some other system of the human or animal. The effect of such treatment of plasma removed from the human or animal and return of the sample containing the partially or completely delipidated infectious organism to the human or animal causes a net decrease in the concentration and infectivity of the infectious organism contained within the vascular system of the human or animal. In this manner, the load or concentration of the infectious organism, whether it is a virus, a bacteria or some other infectious organism, for example, a protozoa or a mold, is reduced. In this continuous mode of operation, the method of the present invention is employed to treat body fluids in a continuous manner, while the human or animal is connected to a system for such treatment.

Another use of the method of the present invention is in a discontinuous or batch mode. In this embodiment, the human or animal is not connected to a device for processing bodily fluids with the method of the present invention. In a discontinuous mode of operation, the present invention employs a fluid, for example, a plasma sample or a sample of lymphatic fluid or follicular fluid, which has previously been obtained from a human or animal. A sample may be contained within a blood bank, or may have simply been removed from a human or animal prior to application of the method. A sample may be a reproductive fluid or any fluid used in the process of artificial insemination or in vitro fertilization. Alternatively, the sample may be one which is not obtained directly from a human or animal but may be cell culture fluid or some other fluid containing a potentially infectious organism. In this mode of operation, this sample is treated with the method of the present invention to produce a new sample which contains partially or completely delipidated infectious organisms. One embodiment of this mode of the present invention is to treat plasma samples previously obtained from animals or humans and stored in a blood bank for subsequent transfusion. These samples may be treated with the method of the present invention to minimize or eliminate transmission of infectious disease, such as HIV, hepatitis, cytomegalovirus, staphylococcus, streptococcus, enterococcus, or meningococcus, from the biological sample.

Delipidation of an infectious organism can be achieved by various means. A batch method can be used for fresh or stored biological fluids, for example, fresh frozen plasma. In this case a variety of the described organic solvents or mixtures thereof can be used for viral inactivation. Extraction time depends on the solvent or (mixed solvent) and the mixing procedure employed.

A continuous method may also be employed for delipidation of an infectious organism, using for example, a device as described in U.S. Patents 4,895,558 or 5,744,038.

### Vaccine Production

The partially or substantially delipidated infectious organism, or components thereof, is combined with a pharmaceutically acceptable carrier to make a composition comprising a vaccine. This vaccine composition is optionally combined with an immunostimulant and administered to an animal or a human. It is to be understood that vaccine compositions may contain more than one partially or substantially delipidated infectious organism or components thereof, in order to provide protection against more than one disease after vaccination. Such combinations may be selected according to the desired immunity. For example, a combination may be HIV and hepatitis, or influenza and hepatitis. The remaining particles of the organism are retained in the delipidated biological fluid, and when reintroduced into the animal or human are presumably taken ingested by phagocytes. The number of particles isolated and affected by the delipidation treatment is determined by counting the particles before and after treatment.

### Administration of Vaccine Produced With the Method of the Present Invention

When a delipidated organism is administered to an animal or a human, it is typically combined with a pharmaceutically acceptable carrier to produce a vaccine, and optionally combined with an immunostimulant as known to one of ordinary skill in the art.

The vaccine formulations may conveniently be presented in unit dosage form and may be prepared by conventional pharmaceutical techniques. Such techniques include the step of bringing into association the active ingredient and the pharmaceutical carrier(s) or excipient(s). In general, the formulations are prepared by uniformly and intimately bringing into association the active ingredient with liquid carriers. Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example, sealed ampules and vials, and may be stored in a freeze-dried (lyophilized) condition requiring only the addition of the sterile liquid carrier, for example, water for injections, immediately prior to use. Extemporaneous injection solutions and suspensions may be prepared from sterile powders, granules and tablets commonly used by one of ordinary skill in the art.

Preferred unit dosage formulations are those containing a dose or unit, or an appropriate fraction thereof, of the administered ingredient. It should be understood that in addition to the ingredients, particularly mentioned above, the formulations of the present invention may include other agents commonly used by one of ordinary skill in the art.

The vaccine may be administered through different routes, such as oral, including buccal and sublingual, rectal, parenteral, aerosol, nasal, intramuscular, subcutaneous, intradermal, and topical. The vaccine of the present invention may be administered in different forms, including but not limited to solutions, emulsions and suspensions, microspheres, particles, microparticles, nanoparticles, and liposomes. It is expected that from about 1 to 5 dosages may be required per immunization regimen. Initial injections may range from about 1 mg to 1 gram, with a preferred range of about 10 mg to 800 mg, and a more preferred range of from approximately 25 mg to 500 mg. Booster injections may range from 1 mg to 1 gram, with a preferred range of approximately 10 mg to 750 mg, and a more preferred range of about 50 mg to 500 mg.

The volume of administration will vary depending on the route of administration. Intramuscular injections may range from about 0.1 ml to 1.0 ml.

The vaccines of the present invention may be administered before, during or after an infection. In one embodiment, the viral load (one or more viruses) of a human or an animal may be reduced by delipidation treatment of the plasma and the same individual may receive a vaccine directed to the one or more viruses, thereby stimulating the immune system to fight the virus that remains in the individual.

The vaccine may be stored at temperatures of from about 4°C to -100°C. The vaccine may also be stored in a lyophilized state at different temperatures including room temperature. The vaccine may be sterilized through conventional means known to one of ordinary skill in the art. Such means include, but are not limited to filtration, radiation and heat. The vaccine of the present invention may also be combined with bacteriostatic agents, such as thimerosal, to inhibit bacterial growth.

### Vaccination Schedule

The vaccine of the present invention may be administered to human or animals. The optimal time for administration of the vaccine is about one to three months before the initial infection. However, the vaccine may also be administered after initial infection to ameliorate disease progression, or after initial infection to treat the disease.

### Adjuvants

A variety of adjuvants known to one of ordinary skill in the art may be administered in conjunction with the protein in the vaccine composition. Such adjuvants include, but are not limited to the following: polymers, co-polymers such as polyoxyethylene-polyoxypropylene copolymers, including block co-polymers; polymer P1005; monotide ISA72; Freund's complete adjuvant (for animals); Freund's incomplete adjuvant; sorbitan monooleate; squalene; CRL-8300 adjuvant; alum; QS 21, muramyl dipeptide; trehalose; bacterial extracts, including mycobacterial extracts; detoxified endotoxins; membrane lipids; or combinations thereof.

It will be appreciated that other embodiments and uses will be apparent to those skilled in the art and that the invention is not limited to these specific illustrative examples.

### EXAMPLE 1

### Delipidation of serum produces inactivation of Duck Hepatitis B virus (DHBV)

A standard duck serum pool (Camden) containing 10⁶ ID₅₀ doses of DHBV was used. ID₅₀ is known to one of ordinary skill in the art as the infective dosage (ID) effective to infect 50% of animals treated with the dose. Twenty-one ducklings were obtained from a DHBV negative flock on day of hatch. These ducklings were tested at purchase and shown to be DHBV DNA negative by dot-blot hybridisation.

The organic solvent system was mixed in the ratio of 40% butanol to 60% diisopropyl ether. 4 ml of the mixed organic solvent system was mixed with 2 ml of the standard serum pool and gently rotated for 1 hour at room temperature. The mixture was centrifuged at 400xg for 10 minutes and the lower aqueous phase removed at room temperature. The lower phase was then mixed with an equal volume of diethyl ether and centrifuged as before. The aqueous phase was then removed and mixed with an equal volume of diethyl ether and re-centrifuged. The aqueous phase was removed and residual diethyl ether was removed by airing in a fume cabinet at room temperature for about 1 hour. The delipidate plasma, with or without viral particles was stored at -20°C.

The positive and negative control duck sera were diluted in phosphate buffered saline (PBS). Positive controls: 2ml of pooled serum containing 10⁶ID₅₀ doses of DHBV was mixed with 4 ml of PBS. Negative controls: 2ml of pooled DHBV negative serum was mixed with 4 ml of PBS. Residual infectivity was tested by inoculation of 100µl of either test sample (n=7), negative (n=7) or positive (n=7) control into the peritoneal cavities of day-old ducks. Control were run with DHBV negative serum treated with organic solvents and then mixed with phosphate buffered saline (PBS) and injected into recipient ducks.

One of the positive control ducks died between 4 and 6 days of age and was excluded from further analysis. A further 3 positive control ducks died between 9 and 10 days of age, and two treatment and one negative control died on day 11. It was decided to terminate the experiment. The remaining ducklings were euthanized on day 12 with sodium pentibarbitone, i.v., and their livers removed for DHBV DNA analysis as described by Deva et al (*J Hospital Infection* 33:119-130, 1996). All seven negative control ducks remained DHBV negative. Livers of all six positive control ducks were DHBV positive. All seven test ducks remained negative for DHBV DNA in their liver.

Delipidation of serum using the above solvent system resulted in inactivation of DHBV. None of the ducklings receiving treated serum became infected. Although the experiment had to be terminated on day 12 instead of day 14 all the positive control ducks were positive for DHBV (3/3 were DHBV positive by day 10). This suggests that sufficient time had elapsed for the treated ducks to become DHBV positive in the liver and that the premature ending of the experiment had no bearing on the results.

### EXAMPLE 2

### Inactivation of Cattle Pestivirus (bovine viral diarrhea virus, BVDV), as a Model for Hepatitis C

A standard cattle pestivirus isolate (BVDV) was used in these experiments. This isolate, "Numerella" BVD virus, was isolated in 1987 from a diagnostic specimen submitted from a typical case of 'Mucosal Disease' on a farm in the Bega district of New South Wales, Australia. This virus is non-cytopathogenic, and reacts with all 12 of a panel of monoclonal antibodies raised at the Elizabeth Macarthur Agricultural Institute (EMAI), NSW, Australia, as typing reagents. Therefore, this virus represents a 'standard strain' of Australian BVD viruses.

The Numerella virus was grown in bovine MDBK cells tested free of adventitious viral agents, including BVDV. The medium used for viral growth contained 10% adult bovine serum derived from EMAI cattle, all tested free of BVDV virus and BVDV antibodies. This serum supplement has been employed for years to exclude the possibility of adventitious BVDV contamination of test systems, a common failing in laboratories worldwide that do not take precautions to ensure the test virus is the only one in the culture system. Using these tested culture systems ensured high level replication of the virus and a high yield of infectious virus. Titration of the final viral yield after 5 days growth in MDBK cells showed a titer of 10^{6.8} infectious viral particles per ml of clarified (centrifuged) culture medium.

### 1. Inactivation of Infectious BVDV

100ml of tissue-culture supernatant, containing 10^{6.8} viral particles/ml, was harvested from a 150 cm² tissue-culture flask. The supernatant was clarified by centrifugation (cell debris pelleted at 3000 rpm, 10 min, 4°C) and 10 ml set aside as a positive control for animal inoculation (non-inactivated virus). The remaining 90 ml, containing 10^{7.75} infectious virus, was inactivated using the following protocol. Briefly, 180 ml of butanol:diisopropyl ether (2:1) was added and mixed by swirling. The mixture, in a 500 ml conical flask, was then shaken for 60 min at 30 rpm at room temperature on an orbital shaker. It was then centrifuged for 10 min at 400 x g at 4°C and the organic solvent phase removed and discarded. In subsequent steps, the bottom layer (aqueous phase) was removed from beneath the organic phase, improving yields considerably.

The aqueous phase, after butanol:diisopropyl ether treatment, was washed 4 times with an equal volume of fresh diethyl ether to remove all contaminating traces of butanol. Each time, the flask was swirled to ensure even mixing of the aqueous and solvent phases before centrifugation as above (400 x g, 10 min, 4°C). After 4 washes, the aqueous phase was placed in a sterile beaker covered with a sterile tissue fixed to the beaker with a rubber band to prevent contamination and placed in a fume hood running continuously overnight (16 hr). Subsequent culture of the inactivated material demonstrated no contamination. The fume hood was left running to remove all remaining volatile ether residue from the inactivated viral preparation. It was then stored at 4°C under sterile conditions until inoculated into tissue culture or animals to test for any remaining infectious virus.

### 2. Testing of inactivated BVDV preparation

### 2.1 Tissue-culture inoculation

2 ml of the solvent-inactivated virus preparation, containing an expected about 10^{7.1} viral equivalents, was mixed with 8 ml tissue-culture medium Minimal Eagles Medium (MEM) containing 10% tested-free adult bovine serum and adsorbed for 60 min onto a monolayer of MDBK cells in a 25 cm² tissue-culture flask. As a positive control, 2 ml of non-inactivated virus (containing the same amount of live, infectious virus) was similarly adsorbed on MDBK cells in a 25 cm² tissue-culture flask. After 60 min, the supernatant was removed from both flasks and replaced with normal growth medium (+10% ABS). The cells were then grown for 5 days under standard conditions before the MDBK cells were fixed and stained using a standard immunoperoxidase protocol with a mixture of 6 BVDV-specific monoclonal antibodies (EMAI panel, reactive with 2 different BVD viral proteins).

There were no infected cells in the monolayer of MDBK cells that was inoculated with the organic-solvent treated (inactivated) virus. In contrast, approximately 90% of the cells in the control flask (that was inoculated with non-inactivated BVD virus) were positive for virus as shown by heavy, specific, immunoperoxidase staining. These results showed that, under *in vitro* testing conditions, no infectious virus remained in the inactivated BVDV preparation.

### Animal Inoculation

An even more sensitive *in vivo* test is to inoculate naïve (antibody-negative) cattle with the inactivated-virus preparation. As little as one infectious viral particle injected subcutaneously in such animals is considered to be an infectious-cow dose, given that entry into cells and replication of the virus is extremely efficient for BVDV.

A group of 10 antibody-negative steers (10-12 months of age) were randomly allocated to 3 groups. The first group of 6 steers was used to test whether the BVD virus had been fully inactivated. The same inactivated preparation of BVD described above was used in this example.

Two steers were inoculated with non-inactivated vaccine to act as a positive-control for the vaccine group, while the 2 remaining steers acted as negative "sentinel" animals to ensure there was no natural pestivirus transmission occurring naturally within the vaccinated group of animals. The positive control animals (inoculated with live, infectious virus) each received 5 ml of the non-inactivated viral preparation (the original viral harvest as described above) and were run under separate, quarantined conditions to stop them from infecting other animals when they developed a transient viraemia after infection (normally at 4-7 days after receiving live BVDV virus). Antibody levels were measured in all 10 animals using a validated, competitive ELISA developed at EMAI. This test has been independently validated by CSL Ltd and is marketed by IDEXX Scandinavia in Europe.

The six animals in the first group each received a subcutaneous injection of 4.5ml of the inactivated BVDV preparation, incorporated in a commercial adjuvant. Since each ml of the inactivated preparation contained 10^{6.8} viral equivalents, the total viral load before inactivation was 10^{7.4} tissue culture infectious doses (TCID)₅₀. The positive-control animals received 5 ml each of the non-inactivated preparation, that is, 10^{7.5} TCID₅₀ injected subcutaneously in the same way as for the first group. The remaining two 'sentinel' animals were not given any viral antigens, being grazed with the first group of animals throughout the trial to ensure there was no natural pestivirus activity occurring in the group while the trial took place.

There was no antibody development in any of the vaccinated steers receiving the inactivated BVD virus preparation until a second dose of vaccine was given. Thus, at 2 and 4 weeks after a single dose, none of the 6 steers seroconverted showing that there was no infectious virus left in a total volume of 27 ml of the inactivated virus preparation. This is the equivalent of a total inactivation of 10^{8.2} TCID₅₀. In contrast, there were high levels of both anti-E2 antibodies (neutralizing antibodies) and anti-NS3 antibodies at both 2 and 4 weeks after inoculation in the 2 animals receiving 5 ml each of the viral preparation prior to inactivation. This confirmed the infectious nature of the virus prior to inactivation. These *in vivo* results confirm the findings of the *in vitro* tissue-culture test. The 2 'sentinel' animals remained seronegative throughout showing the herd remained free of natural pestivirus infections.

The panel of monoclonal antibodies used detected host antibodies directed against the major envelope glycoprotein (E2) which is a glycoprotein incorporated in the lipid envelope of the intact virus. The test systems also detected antibodies directed against the non-structural protein, NS3 that is made within cells infected by the virus. This protein has major regulatory roles in viral replication and is not present within the infectious virus. There was no evidence of intact viral proteins present. There was no evidence in the vaccinated cattle that infectious virus was present, indicating all infectious viral particles had been destroyed. All pestiviruses are RNA viruses. Therefore, there was no viral DNA present in the inactivated preparation.

### EXAMPLE 3

### Inactivated BVDV Preparation as a Vaccine in Steers

All six steers that had received an initial dose of 4.5 ml of the inactivated BVDV preparation described in Example 2 were reinjected subcutaneously with a similar dose at 4 weeks after the first priming dose. At this time there were no antibody responses after the single dose. Animals normally react after the second dose. Strong anamnestic responses for anti-E2 antibody levels (equivalent to serum neutralizing antibodies SNT) were observed in 3 of the 6 steers at 2 weeks after the second dose of the inactivated virus. This response was more than 70% inhibition in a competitive ELISA. The remaining 3 animals showed weak antibody responses (23-31 % inhibition).

In contrast to the anti-E2 antibody responses, only one animal developed a strong anti-NS3 antibody response (93% inhibition) at 2 weeks after the second dose of inactivated BVDV. A second animal had a weak anti-NS3 response (29% inhibition) and 4 animals showed no antibody following administration of 2 doses. This was not unexpected since similar responses following administration of inactivated BVDV vaccines have been observed previously. The antibody levels in steers following 2 doses of the inactivated BVDV preparation demonstrate its potential as a vaccine since antiE2 antibody levels were measurable in all 6 vaccinated steers at 2 weeks after the second dose.

### EXAMPLE 4

### Ultrastructural Analysis of Flavivirus Kunjin Virus Particles before and after Delipidation Treatment

Delipidation was conducted with diisopropyl ether (DIPE)/Butanol and DIPE alone for 60 min, 1 min and 30 seconds. Standard ultrastructural immunocytochemical techniques were used. Bovine serum albumin was used as the blocking solution at a concentration of 1% and the antibodies were diluted in this solution and incubated with the samples for 15 min at room temperature. The gold-labeled Protein-A was purchased from Biocell, UK.

There was no infectivity or visible virus particles detected by EM, even after treatment for 30 seconds. No virus particles were observed for the inactivated samples. It is believed that destruction of the virus liquid envelope occurs too rapidly for observation.

However, when using an unpurified treated sample (i.e. infected tissue culture fluid), although no virus particles were present, some of the proteins could be observed ultrastructurally in conjunction with gold-labeled monoclonal antibodies specific to the major envelope viral protein E. The ultrastructural analysis for visualization of particles actually relies on there being a reasonable titer of virus (approx. 10⁶ particles per ml). In an infectivity assay the delipidation treatment reduced the infectivity of the virus and this process appeared to be time dependant. The treatment therefore reduced the titer to a level that was under that for frequent EM visualization and thus suggests the particles were disassembled because none were observed. While not wanting to be bound by the following statement, some particles were still present as shown by infectivity but the longer the treatment the more inactivation, and probable disassembly, occurred.

### EXAMPLE 5

### Delipidafed DHBV Positive Serum as a Vaccine to Prevent DHBV Infection

The efficacy of the delipidation procedure to provide a vaccine against Duck Hepatitis B Virus (DHBV) was examined.

Approximately 16 Pekin cross ducklings were obtained from a DHBV negative flock of ducklings on the day of hatch. The ducklings were tested and determined to be DHBV negative by analysis of DHBV DNA using dot-blot hybridization. The ducks were divided into three groups: Group 1 contained six ducks that received the test vaccine; Group 2 consisted of four ducks vaccinated with glutaraldehyde-inactivated DHBV, this group is termed sham vaccination; Group 3 consisted of six ducks which were vaccinated with phosphate buffered saline (PBS) - these were considered as mock-vaccinated ducks used as control for the vaccination process. Glutaraldehyde inactivation was achieved by fixation with a dilute solution of glutaraldehyde at about 1:250.

### Delipidation Procedure

An organic solvent system was employed to perform delipidation of serum. The solvent system consisted of a ratio of 40% butanol (analytical reagent grade) and 60% diisopropyl ether. This solvent was mixed with serum in a ratio of 2:1. Accordingly, 4ml of the organic solvent was mixed with 2ml of the serum and rotated for 1 hour. This mixture was centrifuged at approximately 400xg for 10 minutes and the aqueous phase was removed. The aqueous phase was then mixed with an equal volume of diethyl ether and centrifuged at 400xg for 10 minutes. Next, the aqueous phase was removed and mixed with an equal volume of diethyl ether and end-over-end rotation at 30 rpm for about 1 hour, and centrifuged at 400xg for 10 minutes. The aqueous phase was removed and the residual diethyl ether was removed through evaporation in a fume cabinet for approximately 10 to 30 minutes. What remained following removal of diethyl ether was considered the treated serum and was used to produce the vaccine. Controls for the delipidation procedure included subjecting the DHBV negative serum to the same delipidation procedure as the DHBV positive serum.

### Vaccine Production

### Test Vaccine:

1^{st} dose - A 40µl aliquot of the delipidated serum was mixed with 1960µl of phosphate buffered saline (PBS).

2^{nd} dose - A 40µl aliquot of the delipidated serum was mixed with 1960µl of PBS and then emulsified in 1000µl of Freunds Incomplete Adjuvant.

3^{rd} dose - A 200µl aliquot of the delipidated serum was mixed with 1800µl of PBS and then emulsified in 1000µl of Freunds Incomplete Adjuvant.

### Sham vaccination or DHBV Serum Control:

1^{st} dose ―A 200µl aliquot of DHBV positive serum pool #4 (20.4.99) was mixed with 300µl of PBS and 100µl of a 2% glutaraldehyde solution (Aidal Plus from Whiteley Chemicals) and incubated for 10 minutes to inactivate the DHBV. A 40µl aliquot of the inactivated serum/PBS mixture was added to 1960µl PBS.

2^{nd} and 3^{rd} dose― A 200µl aliquot of DHBV positive serum pool #4 (20.4.99) was mixed with 300µl of PBS and 100µl Aidal Plus (Whiteley Chemicals) and incubated for 10 minutes to inactivate the DHBV. A 40µl aliquot of the inactivated serum/PBS mixture was added to 1960µl PBS and emulsified in 1000µl Freunds Incomplete Adjuvant.

### Mock vaccination or negative control:

1^{st} dose - PBS

2^{nd} and 3^{rd} dose - A 2000µl aliquot of PBS was emulsified in 1000µl

Freunds Incomplete Adjuvant.

### Experimental Procedure

### Date of hatch: 23.10.00

Vaccination protocol: 1^{st} dose ―Ducks were injected with 200µl of the respective vaccine into the peritoneal cavity on day 8 post hatch. 2^{nd} dose― Ducks were vaccinated with 300µl of the respective vaccine intramuscularly on day 16 post-hatch. 3^{rd} dose― Ducks were vaccinated with 300µl of the respective vaccine intramuscularly on day 22 post hatch.

Ducks were challenged with 1000µl of DHBV positive serum (serum pool 20.1.97) on day 29 post hatch. Serum pool 20.1.97 was shown to have 1.8 x 10¹⁰ genome equivalent (gev)/ml by dot-blot hybridization. One gev is approximately one viral particle.

Ducks were bled prior to vaccination on days 1 and 10, prior to challenge on days 17 and 23, and post challenge on days 37, 43 and 52. Their serum tested for DHBV DNA by dot-blot hybridization as described by Deva *et al.* (1995). Ducks were euthanized on day 58 and their livers removed, the DNA extracted and tested for the presence of DHBV by dot-blot hybridization as described by Deva *et al.* (1995).

### Results

*Test ducks* Five of the 6 test ducks vaccinated with the test vaccine remained negative for DHBV DNA in the serum and liver following challenge. One test duck became positive for DHBV following challenge. *Sham vaccinated ducks* All four of the ducks vaccinated with glutaraldehyde inactivated serum became DHBV positive following challenge with DHBV.

### Mock vaccinated ducks

Five of the 6 mock-vaccinated negative control ducks became DHBV positive following challenge.

The Chi-square analysis was used to compare differences between treatments. Significantly more control ducks (mock vaccinated) became DHBV positive following challenge than the ducks vaccinated with delipidated serum (p<0.05).

Vaccination of ducklings with delipidated DHBV positive serum using the above protocol resulted in prevention of DHBV infection following challenge with DHBV positive serum in 5 of 6 ducklings. This suggests that the delipidated serum vaccine is capable of inducing immunity in vaccinated ducks. In comparison 5 of 6 mock vaccinated and 4 of 4 sham vaccinated ducks became DHBV positive following vaccination suggesting no induction of immunity in these ducks.

It should be understood, of course, that the foregoing relates only to preferred embodiments of the present invention and that numerous modifications or alterations may be made therein without departing from the spirit and the scope of the invention as set forth in the appended claims.

## Claims

1. A method for reducing levels of a lipid-containing infectious organism in an aqueous fluid comprising:
contacting the aqueous fluid containing the lipid-containing infectious organism with a first organic solvent, selected from the group of alcohol having ≥ 5 C atoms, ether, amine, hydrocarbons or a combination thereof or a combination of an alcohol having 1 to 8 C atoms with ether, amine, hydrocarbons or a combination thereof in a ratio wherein the first solvent is present in an amount effective to substantially solubilize the lipid in the infectious organism;
mixing the aqueous fluid and said first solvent;
permitting organic and aqueous phases to separate; and
collecting the aqueous phase containing the infectious organism with reduced lipid content.

2. The method of claim 1, further comprising:
contacting the aqueous phase with a de-emulsifying agent capable of removing the first organic solvent; and,
separating the de-emulsifying agent containing the removed first organic solvent from the contacted aqueous phase.

3. The method according to claim 1 or 2 further comprising adding cells to the aqueous phase containing the infectious organism with reduced lipid content.

4. A method according to claim 3, whereby the fluid is blood, plasma, serum, cerebrospinal fluid, lymphatic fluid, peritoneal fluid, follicular fluid, amniotic fluid, pleural fluid, pericardial fluid or reproductive fluids.

5. A method for making a vaccine comprising:
contacting a lipid-containing infectious organism in an aqueous fluid with a first organic solvent, selected from the group of alcohol having ≥ 5 C atoms, ether, amine, hydrocarbons, surfactants and esters or a combination thereof or a combination of an alcohol having 1 to 8 C atoms with ether, amine, hydrocarbons or a combination thereof;
mixing the aqueous fluid and the first organic solvent for a time sufficient to extract lipid from the lipid-containing infectious organism;
permitting organic and aqueous phases to separate; and collecting the aqueous phase containing the infectious organism with reduced lipid content.

6. The method of claim 5, further comprising:
contacting the aqueous phase with a de-emulsifying agent capable of removing the first organic solvent; and,
separating the de-emulsifying agent and the removed first organic solvent from the contacted aqueous phase.

7. A method according to any of claims 1 to 6, wherein the lipid-containing infectious organism is a virus, bacterium, protozoan or fungus.

8. The method according to claim 7, wherein the infectious organism is a virus and the virus is immunodeficiency virus, hepatitis or pestivirus.

9. The method according to any of claims 1 to 8, wherein the first organic solvent is a combiantion of an alcohol and an ether.

10. The method of claim 9, wherein the ether is C₄ to C₈ ether and the alcohol is C₁ to C₅ alcohol.

11. The method of claim 2 or 6, wherein the de-emulsifying agent is an ether.

12. A vaccine composition obtainable according to any of claims 5 to 11, comprising a substantially delipidated infectious organism and a pharmaceutically acceptable carrier.

13. The vaccine composition of claim 12, further comprising an immuno-stimulant.

14. Use of an organic solvent mixture comprising diisopropyl ether and butanol for removal of lipid from a lipid-containing infectious organism in a fluid.

15. Use of an organic solvent mixture comprising diisopropyl ether and butanol for the preparation of a solvent system for treating blood infected by a lipid-containing infectious organism.

16. Use of an organic solvent system comprising diisopropyl ether and butanol for preparation of a vaccine composition comprising a fluid containing reduced levels of a lipid-containing infectious organism.

17. The use of claim 14 or 16, wherein the fluid is blood, plasma, serum, cerebrospinal fluid, lymphatic fluid, or peritoneal fluid obtained from an animal or a human.

## Patentansprüche

1. Verfahren zur Verringerung von Mengen eines lipidhaltigen infektiösen Organismus in einem wässrigen Fluid, umfassend:
Inkontaktbringen des den lipidhaltigen infektiösen Organismus enthaltenden wässrigen Fluids mit einem ersten organischen Lösungsmittel, ausgewählt aus der Gruppe von Alkoholen mit ≥ 5 C-Atomen, Ether, Amin, Kohlenwasserstoffen oder einer Kombination davon oder eine Kombination eines Alkohols mit 1 bis 8 C-Atomen mit Ether, Amin, Kohlenwasserstoffen oder eine Kombination davon, in einem Verhältnis, worin das erste Lösungsmittel in einer Menge vorliegt, die wirksam ist, das Lipid im infektiösen Organismus im Wesentlichen zu lösen;
Mischen des wässrigen Fluids und des ersten Lösungsmittels;
Zulassen einer Trennung zwischen organischen und wässrigen Phasen; und
Sammeln der wässrigen Phase, die den infektiösen Organismus mit verringertem Lipidgehalt enthält.

2. Verfahren nach Anspruch 1, ferner umfassend:
Inkontaktbringen der wässrigen Phase mit einem deemulgierendem Mittel, das das erste organische Lösungsmittel entfernen kann; und
Abtrennen des deemulgierenden Mittels, das das entfernte erste organische Lösungsmittel enthält, von der kontaktierten wässrigen Phase.

3. Verfahren nach Anspruch 1 oder 2, ferner umfassend das Zugeben von Zellen zu der wässrigen Phase, die den infektiösen Organismus mit verringertem Lipidgehalt enthält.

4. Verfahren gemäß Anspruch 3, wobei das Fluid Blut, Plasma, Serum, Hirnflüssigkeit, Lymphflüssigkeit, Peritonealflüssigkeit, Follikelflüssigkeit, Fruchtwasser, Flüssigkeit aus dem Pleuraraum, perkardiale Flüssigkeit oder eine Reproduktionsflüssigkeit ist.

5. Verfahren zur Herstellung eines Impfstoffs, umfassend:
Inkontaktbringen eines lipidhaltigen infektiösen Organismus in einem wässrigen Fluid, mit einem ersten organischen Lösungsmittel, ausgewählt aus der Gruppe von Alkoholen mit ≥ 5 C-Atomen, Ether, Amin, Kohlenwasserstoffen, oberflächenaktiven Mitteln und Estern oder eine Kombination davon oder eine Kombination eines Alkohols mit 1 bis 8 C-Atomen mit Ether, Amin, Kohlenwasserstoffen oder eine Kombination davon;
Mischen des wässrigen Fluids und des ersten organischen Lösungsmittels für eine Zeitspanne, die ausreicht, Lipid aus dem lipidhaltigen infektiösen Organismus zu extrahieren;
Zulassen einer Trennung zwischen organischen und wässrigen Phasen; und
Sammeln der wässrigen Phase, die den infektiösen Organismus mit verringertem Lipidgehalt enthält.

6. Verfahren nach Anspruch 5, ferner umfassend:
Inkontaktbringen der wässrigen Phase mit einem deemulgierendem Mittel, das das erste organische Lösungsmittel entfernen kann; und
Abtrennen des deemulgierenden Mittels und des entfernten ersten organischen Lösungsmittels von der kontaktierten wässrigen Phase.

7. Verfahren nach einem der Ansprüche 1 bis 6, wobei der lipidhaltige infektiöse Organismus ein Virus, ein Bakterium, ein tierischer Einzeller oder ein Pilz ist.

8. Verfahren nach Anspruch 7, wobei der infektiöse Organismus ein Virus ist und der Virus ein Immunschwächevirus, Hepatitis oder Pestivirus ist.

9. Verfahren nach einem der Ansprüche 1 bis 8, wobei das erste organische Lösungsmittel eine Kombination aus einem Alkohol und einem Ether ist.

10. Verfahren nach Anspruch 9, wobei der Ether ein C₄ bis C₈ Ether und der Alkohol ein C₁ bis C₅ Alkohol ist.

11. Verfahren nach Anspruch 2 oder 6, wobei das deemulgierende Mittel ein Ether ist.

12. Impfstoffzusammensetzung erhältlich nach einem der Ansprüche 5 bis 11, umfassend einen im Wesentlichen entfetteten Organismus und einen pharmazeutisch annehmbaren Träger.

13. Impfstoffzusammensetzung nach Anspruch 12, ferner umfassend ein immunstimulierendes Mittel.

14. Verwendung eines organischen Lösungsmittelgemischs, umfassend Diisopropylether und Butanol zur Entfernung von Lipid aus einem lipidhaltigem infektiösem Organismus in einem Fluid.

15. Verwendung eines organischen Lösungsmittelgemischs, umfassend Diisopropylether und Butanol zur Herstellung eines Lösungsmittelsystems zur Behandlung von Blut, das mit einem lipidhaltigen infektiösen Organismus infiziert ist.

16. Verwendung eines organischen Lösungsmittelsystems, umfassend Diisopropylether und Butanol zur Herstellung einer Impfstoffzusammensetzung umfassend ein Fluid, das verringerte Mengen eines lipidhaltigen infektiösen Organismus enthält.

17. Verwendung nach Anspruch 14 oder 16, wobei das Fluid Blut, Plasma, Serum, Hirnflüssigkeit, Lymphflüssigkeit oder Peritonealflüssigkeit ist, die/das von einem Tier oder einem Menschen erhalten wird.

## Revendications

1. Procédé pour réduire les niveaux d'un organisme infectieux contenant des lipides dans un fluide aqueux, comprenant :
- la mise en contact du fluide aqueux contenant l'organisme infectieux contenant des lipides avec un premier solvant organique sélectionné dans le groupe comprenant un alcool contenant 5 ou plus de 5 atomes de C, un éther, une amine, des hydrocarbures ou une combinaison de ceux-ci ou une combinaison d'un alcool contenant 1 à 8 atomes de C avec un éther, une amine, des hydrocarbures ou une combinaison de ceux-ci dans un rapport dans lequel le premier solvant est présent en une quantité efficace pour solubiliser sensiblement les lipides présents dans l'organisme infectieux;
- le mélange du fluide aqueux et du premier solvant;
- la séparation naturelle des phases organique et aqueuse; et
- la collecte de la phase aqueuse contenant l'organisme infectieux présentant une teneur en lipides réduite.

2. Procédé selon la revendication 1, comprenant en outre:
- la mise en contact de la phase aqueuse avec un agent désémulsifiant capable d'éliminer le premier solvant organique; et
- la séparation de l'agent désémulsifiant contenant le premier solvant organique éliminé de la phase aqueuse mise en contact.

3. Procédé selon la revendication 1 ou 2,
comprenant en outre l'addition de cellules à la phase aqueuse contenant l'organisme infectieux présentant une teneur en lipides réduite.

4. Procédé selon la revendication 3,
selon lequel le fluide est du sang, du plasma, du sérum, du liquide céphalorachidien, du liquide lymphatique, du liquide péritonéal, du liquide folliculaire, du liquide amniotique, du liquide pleural, du liquide péricardique ou des liquides de reproduction.

5. Procédé pour fabriquer un vaccin, comprenant:
- la mise en contact d'un organisme infectieux contenant des lipides dans un fluide aqueux avec un premier solvant organique sélectionné dans le groupe comprenant un alcool contenant 5 ou plus de 5 atomes de C, un éther, une amine, des hydrocarbures, des tensioactifs et des esters, ou une combinaison de ceux-ci, ou une combinaison d'un alcool contenant de 1 à 8 atomes de C avec un éther, une amine, des hydrocarbures ou une combinaison de ceux-ci;
- le mélange du fluide aqueux et du premier solvant organique pendant une durée suffisante pour extraire les lipides hors de l'organisme infectieux contenant des lipides;
- la séparation naturelle des phases organique et aqueuse; et
- la collecte de la phase aqueuse contenant l'organisme infectieux présentant une teneur en lipides réduite.

6. Procédé selon la revendication 5, comprenant en outre:
- la mise en contact de la phase aqueuse avec un agent désémulsifiant capable d'éliminer le premier solvant organique; et
- la séparation de l'agent désémulsifiant contenant le premier solvant organique éliminé de la phase aqueuse mise en contact.

7. Procédé selon l'une quelconque des revendications 1 à 6,
selon lequel l'organisme infectieux contenant des lipides est un virus, une bactérie, un protozoaire ou un champignon.

8. Procédé selon la revendication 7,
selon lequel l'organisme infectieux est un virus, et le virus est un virus d'immunodéficience, de l'hépatite ou de la peste.

9. Procédé selon l'une quelconque des revendications 1 à 8,
selon lequel le premier solvant organique est une combinaison d'un alcool et d'un éther.

10. Procédé selon la revendication 9,
selon lequel l'éther est un éther C₄ à C₈ et l'alcool est un alcool C₁ à C₅.

11. Procédé selon la revendication 2 ou 6,
selon lequel l'agent désémulsifiant est un éther.

12. Composition de vaccin pouvant être obtenue selon l'une quelconque des revendications 5 à 11,
comprenant un organisme infectieux essentiellement débarrassé de ses lipides et un porteur acceptable sur le plan pharmaceutique.

13. Composition de vaccin selon la revendication 12,
contenant en outre un immuno-stimulant.

14. Utilisation d'un mélange de solvant organique contenant de l'éther diisopropylique et du butanol pour l'élimination des lipides hors d'un organisme infectieux contenant des lipides dans un fluide.

15. Utilisation d'un mélange de solvant organique contenant de l'éther diisopropylique et du butanol pour la préparation d'un système de solvant pour traiter du sang infecté par un organisme infectieux contenant des lipides.

16. Utilisation d'un système de solvant organique contenant de l'éther diisopropylique et du butanol pour la préparation d'une composition de vaccin comprenant un fluide contenant des niveaux réduits d'un organisme infectieux contenant des lipides.

17. Utilisation selon la revendication 14 ou 16,
selon laquelle le fluide est du sang, du plasma, du sérum, du liquide céphalorachidien, du liquide lymphatique ou du liquide péritonéal prélevé chez un animal ou chez un être humain.
